(19) Europäisches Patentamt
European Patent Office
Office européen des brevets

(11) Veröffentlichungsnummer: **0 009 796**
**B1**

(12) # EUROPÄISCHE PATENTSCHRIFT

(45) Veröffentlichungstag der Patentschrift: **04.11.81**

(51) Int. Cl.³: **C 07 C 69/712,** C 07 C 67/11

(21) Anmeldenummer: **79103731.0**

(22) Anmeldetag: **01.10.79**

(54) **Verfahren zur Herstellung von (Phenoxy- bzw. Benzyl)-phenoxypropionsäuremethylestern.**

(30) Priorität: **04.10.78 DE 2843184**

(43) Veröffentlichungstag der Anmeldung: **16.04.80 Patentblatt 80/8**

(45) Bekanntmachung des Hinweises auf die Patenterteilung: **04.11.81 Patentblatt 81/44**

(84) Benannte Vertragsstaaten: **AT BE CH DE FR GB IT NL**

(56) Entgegenhaltungen: **US-A-1 924 575**

(73) Patentinhaber: **HOECHST AKTIENGESELLSCHAFT, Postfach 80 03 20, D-6230 Frankfurt/Main 80 (DE)**

(72) Erfinder: **Koch, Manfred, Dr., Kreuzheck 2, D-6239 Eppstein/Taunus (DE)**
Erfinder: **Mildenberger, Hilmar, Dr., Fasanenstrasse 24, D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 009 796 B1

BUNDESDRUCKEREI BERLIN

# Verfahren zur Herstellung von (Phenoxy- bzw. Benzyl)-phenoxipropionsäuremethylestern

Die vorliegende Erfindung betrifft ein Verfahren zur Herstellung von (Phenoxy- bzw. Benzyl)-phenoxipropionsäuremethylestern der Formel I

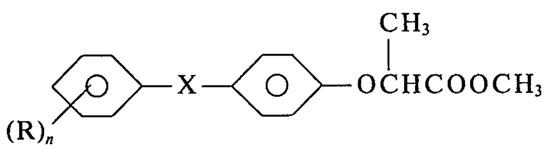

(I)

worin die Reste R gleich oder verschieden sind und Alkyl, Halogen, $CF_3$ oder $NO_2$ bedeuten; n 1 oder 2 ist und X Sauerstoff oder die Gruppe $-CH_2-$ bedeutet, das dadurch gekennzeichnet ist, daß man wasserfreie Alkalisalze von entsprechend substituierten (Phenoxy- bzw. Benzyl-)phenoxipropionsäuren mit im wesentlichen halbmolaren Mengen Dimethylsulfat in einem inerten unpolaren Lösungsmittel bei Reaktionstemperaturen von mindestens 120°C, vorzugsweise von 120–150°C, reagieren läßt.

Die Verbindungen der Formel I sind hochwirksame, selektive Grasherbizide und aus den deutschen Offenlegungsschriften 2 223 894, 2 417 487, 2 433 067, 2 601 548 und 2 652 584 bekannt.

Die Alkylierung von Carbonsäuresalzen mit Dimethylsulfat zu Carbonsäuremethylestern ist an sich bekannt. Im allgemeinen wird dabei das Dimethylsulfat in äquimolarer Menge oder im Überschuß bezogen auf das Carbonsäuresalz eingesetzt (Houben-Weyl, Band VIII, S. 542 (1952); Ullmann 4. Auflage, Band 10, S. 103 (1975); Monatshefte für Chemie 99, 103 (1968); C. A. 49, 11594h (1953). Die dabei erzielten Ausbeuten von 82 bis 96% der Theorie sind jedoch für technische Zwecke wenig befriedigend. Die erwähnte Verfahrensweise hat ferner den Nachteil, daß die Hälfte des Dimethylsulfats als Methylschwefelsäure verloren geht; außerdem hinterbleiben stets Reste von unverändertem Dimethylsulfat, die wegen der Gefahr einer Esterhydrolyse nicht — wie beispielsweise bei der Phenolverätherung mit Dialkylsulfaten — mittels Alkalilaugen nach Beendigung der Reaktion entfernt werden können. Wegen der Toxizität von Dimethylsulfat ist die Methode daher insbesondere für großtechnische Prozesse nicht geeignet.

Es hat daher nicht an Versuchen gefehlt, auch das bei der Reaktion entstehende Methylschwefelsäuresalz entsprechend dem nachstehenden Schema für die Veresterung nutzbar zu machen und dadurch die erforderliche Menge Dimethylsulfat zu vermindern:

$$2\ RCOOMe + (CH_3O)_2SO_2 \longrightarrow RCOOCH_3 + RCOOMe + CH_3OSO_3Me$$

$$\longrightarrow 2\ RCOOCH_3 + Me_2SO_4 \quad (Me = \text{einwertiges Kation})$$

Dies gelingt jedoch laut Houben-Weyl (a. a. O.) nur »unter Druck und höherer Temperatur« (nähere Angaben fehlen). Ullmann a. a. O. weist im gleichen Zusammenhang auf die Notwendigkeit einer langen Nachreaktion bei höherer Temperatur hin. Ein in Ann. 340, 246 (1905) beschriebenes Verfahren zur Alkylierung von Benzoesäuresalzen mit der halbmolaren Menge Dimethylsulfat erfordert Reaktionstemperaturen von 180–260°C und liefert den Benzoesäuremethylester in einer Ausbeute von nur 75% der Theorie. Die Alkylierung von Benzoesäurekaliumsalz mit einer molaren Menge Methylschwefelsäurekaliumsalz bei 210–250°C ergibt eine noch geringere Ausbeute.

Die in C. A. 66, 30 233h (1967) beschriebene Alkylierung von Fettsäurenatriumsalzen mit der halben Molmenge Dimethylsulfat in Wasser läuft zwar bereits bei einer Reaktionstemperatur von 150°C ab, benötigt, aber eine Reaktionszeit von mindestens 10 Stunden und liefert nur eine Ausbeute von etwa 85% Fettsäuremethylester (vgl. Ullmann a. a. O.).

Es ist daher als äußerst überraschend zu bezeichnen, daß bei Verwendung der Ausgangsstoffe gemäß der Erfindung die Umsetzung mit der halbmolaren Menge Dimethylsulfat bereits bei wesentlich niedrigeren Reaktionstemperaturen in wesentlich kürzerer Zeit und in nahezu quantitativer Ausbeute zu den Verbindungen der Formel I führt.

Die Reaktion verläuft nach folgender Gleichung (Me steht hierbei für ein einwertiges Kation):

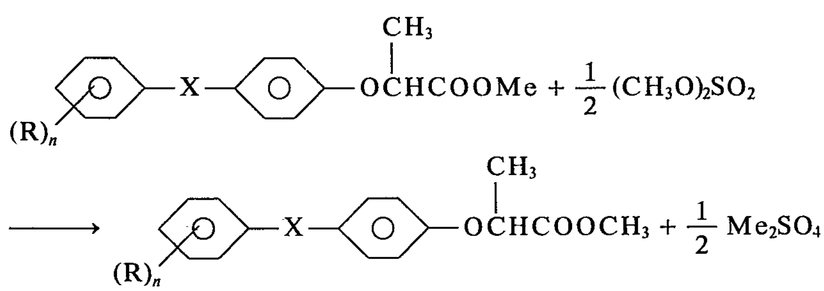

(II)

(I)

Zur Ausführung der Umsetzung werden die wasserfreien Alkalisalze der Formel II in einem inerten unpolaren Lösungsmittel, vorzugsweise Kohlenwasserstoffen wie Benzol, Toluol, Xylol oder Cumol oder Halogenkohlenwasserstoffen wie Chlorbenzol und Dichlorbenzol, bevorzugt in Xylol suspendiert und mit Dimethylsulfat versetzt.

Während die Umsetzung von Dimethylsulfat zu Methylschwefelsäuresalz spontan erfolgt und auch bei niedrigeren Reaktionstemperaturen ausreichend schnell verläuft, ist zur Umsetzung des Methylschwefelsäuresalzes zu Natriumsulfat eine Reaktionstemperatur von mindestens 120°C erforderlich. Bei niedriger siedenden Lösungs-, bzw. Verdünnungsmitteln ist eine Erhöhung der Siedetemperatur durch Anwendung von äußerem Druck notwendig. Die zur quantitativen Umsetzung von Salzen der Formel II notwendige Reaktionszeit ist abhängig von der Reaktionstemperatur; sie beträgt bei einer Reaktionstemperatur von 135°C etwa 1 Stunde. Nach beendeter Umsetzung reagiert die Reaktionsmischung neutral. Anorganische Salze können direkt oder nach Ansäurern mit wenig Mineralsäure durch Auswaschen mit Wasser bzw. durch Filtrieren oder Zentrifugieren entfernt werden.

Bei Verwendung der halbmolaren Menge Dimethylsulfat (bezogen auf das eingesetzte Salz) erhält man eine Umsetzung von 99,3–99,5% der Theorie. Wendet man Dimethylsulfat in geringem Überschuß (5%) bezogen auf die halbmolare Menge an eingesetztem Salz der Formel II an, wird ein Umsatz von über 99,8% erzielt. Zur sicheren Durchführung kann ein Überschuß bis zu 10% verwendet werden.

Durch folgende Beispiele wird das Verfahren erläutert:

Beispiel 1

Methyl-2-[4'-(4''-trifluormethylphenoxi)-phenoxi]-propionat

348 g wasserfreies Natrium-2-[4'-(4''-Trifluormethylphenoxi)-phenoxi]-propionat, suspendiert in 1000 ml Xylol, werden bei 130°C mit 63 g Dimethylsulfat innerhalb von 5 Minuten versetzt. Nach Zugabe wird bei 135°C 1 Stunde nachgerührt, anschließend wird auf 85°C abgekühlt, die Reaktionsmischung bei dieser Temperatur mit 200 ml Wasser und 0,5 ml $H_3PO_4$ versetzt und zur Abtrennung der anorganischen Salze 10 Minuten gerührt. Nach Abtrennung der Wasserphase wird Xylol abdestilliert. Man erhält 340 g Methyl-2-[4'-(4''-trifluormethylphenoxi)-phenoxi]-propionat vom Sp. 142°/0,07 mbar. Der Gehalt an reinem Endprodukt beträgt laut Gaschromatogramm 99,4%, der Restgehalt an nichtumgesetzter Säure 0,6% entsprechend einer Ausbeute von 99,4%.

Beispiel 2

Methyl-2-[4'-(2'',4''-dichlorphenoxi)-phenoxi]-propionat

Zu 349 g wasserfreiem Natrium-2-[4'-(2'',4''-dichlorphenoxi)-phenoxi]-propionat in 1000 ml Xylol gibt man bei 130°C innerhalb 5 Minuten 66,2 g Dimethylsulfat. Nach Zugabe wird bei 138°C 1 Stunde gerührt, anschließend auf 90°C abgekühlt und die Reaktionsmischung mit 200 ml Wasser versetzt.

Bei 85°C wird 10 Minuten intensiv gerührt, anschließend wird die Wasserphase abgetrennt und Xylol abdestilliert. Man erhält 341 g Methyl-2-[4'-(2'',4''-dichlorphenoxi)-phenoxi]-propionat vom Sp. 168°/0,07 mbar. Der Gehalt an reinem Endprodukt beträgt laut Gaschromatogramm 99,9%, entsprechend einer Ausbeute von 99,9%.

In analoger Weise und Ausbeute wurden erhalten:

Methyl-2-[4'-(4''-chlorphenoxi)-phenoxi]-propionat,
Sp. 154°/0,2 mbar,
Methyl-2-[4'-(4''-chlorbenzyl)-phenoxi]-propionat,
Sp. 144–148°/0,09 mbar,
Methyl-2-[4'-(2'',4''-dichlorbenzyl)-phenoxi]-propionat,
Sp. 158–161°/0,07 mbar,
Methyl-2-[4'-(2''-chlor-4''-trifluormethylphenoxi)-phenoxi]-propionat,
Sp. 155°/0,07 mbar,
Methyl-2-[4'-(4''-brom-2''-chlorphenoxi)-phenoxi]-propionat,
Sp. 195–198°/0,5 mbar,
Methyl-2-[4'-(4''-chlor-2''-nitrophenoxi)-phenoxi]-propionat,
Sp. 224–300°/4 mbar.

**Patentansprüche**

1. Verfahren zur Herstellung von (Phenoxi- bzw. Benzyl)-phenoxipropionsäuremethylestern der allgemeinen Formel I

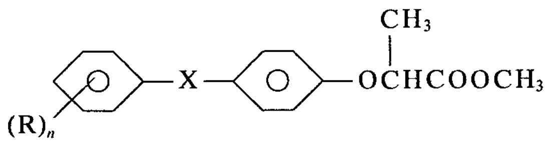

(I)

worin die Reste R gleich oder verschieden sind und Alkyl, Halogen, $CF_3$ oder $NO_2$ bedeuten, n 1 oder 2 ist und X Sauerstoff oder die Gruppe $-CH_2-$ bedeutet, dadurch gekennzeichnet, daß man wasserfreie Alkalisalze von entsprechend substituierten (Phenoxi-bzw. Benzyl-)phenoxipropionsäuren mit im wesentlichen halbmolaren Mengen Dimethylsulfat in einem inerten unpolaren Lösungsmittel bei Reaktionstemperaturen von mindestens 120° C reagieren läßt.

2. Verfahren gemäß Anspruch 1, dadurch gekennzeichnet, daß man Umsetzungstemperaturen von 120 – 150° C anwendet.

3. Verfahren gemäß Ansprüchen 1 und 2, dadurch gekennzeichnet, daß man als Lösungsmittel Kohlenwasserstoffe oder Halogenkohlenwasserstoffe verwendet.

**Claims**

1. Process for the manufacture of (phenoxy or benzyl)-phenoxypropionic acid methyl esters of the general formula

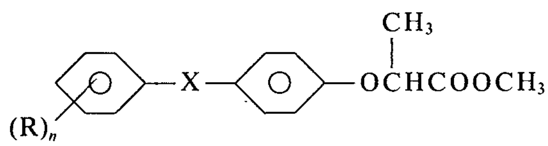

(I)

in which the radicals R, which are identical or different, are alkyl, halogen, $CF_3$ or $NO_2$; n is 1 or 2 ans X is oxygen or the group $-CH_2-$, which comprises reacting anhydrous alkali metal salts of correspondingly substituted (phenoxy or benzyl)-phenoxypropionic acids with an approximately semimolar amount of dimethyl sulfate in a inert non-polar solvent at a temperature of at least 120° C.

2. The process of Claim 1, weherin the reaction is carried out at a temperature of from 120 to 150° C.

3. The process of Claim 1 and 2, wherein hydrocarbons or halohydrocarbons are used as solvent.

**Revendications**

1. Procédé pour préparer des esters méthyliques d'acides (phénoxy ou benzyl)-phénoxy-propioniques répondant à la formule I

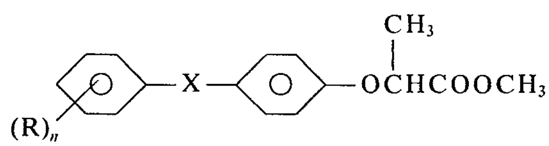

(I)

dans laquelle les symboles R représentent chacun, indépendamment l'un de l'autre, un radical alkyle, un halogène, $CF_3$ ou $NO_2$, n est égal à 1 ou à 2 et X représente l'oxygène ou le groupe $-CH_2-$, procédé caractérisé en ce qu'on fait réagir à une température d'au moins 120° C des sels anhydres de métaux alcalins d'acides (phénoxy ou benzyl)-phénoxypropioniques, substitués de façon correspondante, avec des quantités à peu près hémimolaires de sulfate de diméthyle, dans un solvant inerte non polaire.

2. Procédé selon la revendication 1 caractérisé en ce qu'on effectue la réaction à des températures de 120 à 150° C.

3. Procédé selon l'une des revendications 1 et 2 caractérisé en ce qu'on utilise, comme solvants des hydrocarbures ou des hydrocarbures halogénés.